(19) Europäisches Patentamt
European Patent Office
Office européen des brevets

(11) Veröffentlichungsnummer: **0 299 424 B1**

(12) # EUROPÄISCHE PATENTSCHRIFT

(45) Veröffentlichungstag der Patentschrift: **20.10.93**

(21) Anmeldenummer: **88111119.9**

(22) Anmeldetag: **12.07.88**

Verbunden mit 88907055.3/0324023
(europäische
Anmeldenummer/Veröffentlichungsnummer)
durch Entscheidung vom 15.10.90.

(51) Int. Cl.5: **A61K 31/21**, A61K 31/44, A61K 31/445, A61K 31/535, A61K 31/35, C07C 331/00, A61K 35/78

(54) **Thiosulfinsäurederivate, und die Verwendung von Thiosulfinsäurederivaten zur Herstellung von Arzneimitteln für die Behandlung von Entzündungserkrankungen.**

(30) Priorität: **14.07.87 DE 3723248**

(43) Veröffentlichungstag der Anmeldung:
**18.01.89 Patentblatt 89/03**

(45) Bekanntmachung des Hinweises auf die Patenterteilung:
**20.10.93 Patentblatt 93/42**

(84) Benannte Vertragsstaaten:
**AT BE CH DE ES FR GB GR IT LI LU NL SE**

(56) Entgegenhaltungen:
**EP-A- 0 153 881**
**DE-A- 3 305 951**
**GB-A- 1 109 535**
**US-A- 2 508 745**

**N.S.DHALLA et al., "MYOCARIDIAL ISCHEMIA", Proceedings of a Satellite Symposium of the 30th Internatl. Physiological Congress, Winnipeg, MB (CD), 08-11 Juli 1986, M.Nijhoff Publ., Dordrecht (NL); P.R.MAYEUX et al., Seiten 339-353&NUM;**

(73) Patentinhaber: **BOEHRINGER MANNHEIM GMBH**

**D-68298 Mannheim(DE)**

(72) Erfinder: **Wagner, Hildebert, Prof.**
**Nelkenweg 9**
**D-8211 Breitbrunn/Chiemsee(DE)**
Erfinder: **Dorsch, Walter, Dr. med.**
**Schönstrasse 73 b**
**D-8000 München 90(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

J. ETHNOPHARMACOL., Band 6, 1982, Elsevier Sequoia (NL); E.M.CAPPELLETTI et al., Seiten 161-190&NUM;

THROMBOSIS RESEARCH, Band 44, 1986, Pergamon Journals Ltd. (US); S.FAZAL MOHAMMED et al., Seiten 793-806&NUM;

SCI. AM., Band 252, Nr. 3, 1985; E.BLOCK, Seiten 114-119&NUM;

INT. ARCH. ALLERGY APPL. IMMUN., Band 82, Nrs. 3/4, 1987; W.DORSCH et al., Seiten 535-536&NUM;

BIOCHEMICAL PHARMACOLOGY, Band 29, 1980, Pergamon Press Ltd. (GB); J.Y.VANDERHOEK et al., Seiten 3169-3173&NUM;

THE MERCK MANUAL OF DIAGNOSIS AND THERAPY, Ed. R. Berkow et al., 15. Aufl., 1987, Merck & Co. Ltd., Rahway, NJ (US); Seiten 2506-2516&NUM;

## Beschreibung

Die Erfindung betrifft die Verwendung von Thiosulfinsäurederivaten zur Behandlung von Erkrankungen, bei denen entzündliche Vorgänge im weitesten Sinn beteiligt sind. Zu diesen Erkrankungen sind insbesondere die Erkrankungen des rheumatischen Formenkreises, allergische Erkrankungen, Asthma, Entzündungen, die nicht den vorhin genannten zugerechnet werden, sowie thrombotische Erkrankungen zu zählen.

Es ist allgemein bekannt, daß Inhaltsstoffe verschiedener Pflanzen aus der Familie der Liliaceae, insbesondere der Alliumarten, medizinisch vorteilhafte Wirkungen besitzen. Der modernen Medizin seit langem bekannt sind die antibakteriellen, antimykotischen und zytostatischen Wirkungen des Knoblauchs; auch senkt er den Blutdruck sowie den Zucker- und Lipidspiegel.

Aus der US-PS 2 554 088 ist bekannt, daß Knoblauchextrakte eine antibiotische Wirkung besitzen und daß die antibiotische Wirkung der Verbindung Allyldisulfidoxid zuzuschreiben ist. Weiterhin ist aus der US-PS 2 508 745 bekannt, daß bestimmte Thiosulfinsäureester antibakterielle und fungizide Wirkungen aufweisen.

Es wurde nun gefunden, daß Thiosulfinsäurederivate der allgemeinen Formel I

$R_1$ - S (O) - S - $R_2$

in der $R_1$ und $R_2$ gleich oder verschieden sein können und einen gegebenenfalls einfach oder mehrfach substituierten Alkyrest, Arylrest, Aralkylrest, alicyclischen Rest, oder heterocyclischen Rest bedeuten, die entweder - soweit sie Zwiebelinhaltsstoffe sind - aus dem Saft gepreßter Zwiebeln (All. cepa) extrahiert oder auf chemischem Weg hergestellt werden können, generelle entzündungshemmende Eigenschafteen sowohl in vitro als auch in vivo aufweisen.

Die Verbindungen der allgemeinen Formel I sind zum Teil neue Stoffe, zum Teil gehören ihre Struktur aber noch keine pharmakologische Verwendbarkeit zum Stand der Technik (siehe z. B. Rec. Trav. Chim. Pays-Bas 73, 129 (1954). Allen gemeinsam ist, daß diese Verbindungen eine ausgezeichnete antiasthmatische, entzündungshemmende, antiallergische, analgetische und auch antipyretische Wirkung besitzen. Sie sind auch zur Verhinderung von Gewebeabstoßreaktionen geeignet.

Erfindungsgemäß werden daher Thiosulfinsäurederivate der allgemeinen Formel

$$R_1 - \overset{\overset{O}{||}}{S} - S - R_2 \qquad (I),$$

worin $R_1$ und $R_2$ die oben angegebenen Bedeutungen haben gegebenenfalls in Verbindung mit einem pharmakologisch verträglichen Träger zur Verwendung bei der Behandlung bzw. zur Verwendung zur Herstellung von Arzneimitteln zur Behandlung einer Erkrankung des oben genannten Formenkreises zur Verfügung gestellt.

Unter einem Alkylrest sind geradkettige, verzweigte, gesättigte und ungesättigte Alkylreste mit 1 bis 6 Kohlenstoffatomen, zum Beispiel ein Methyl-, Ethyl-, n-Propyl-, Isopropyl-, Alkyl-, 1-Propenylrest, Butyl-, Pentyl- und Hexylrest zu verstehen. Besonders bevorzugt sind Methyl-, Ethyl-, n-Propyl-, Allyl- und 1-Propenylreste.

Unter einem Arylrest oder dem Arylteil einer Aralkylgruppe ist ein aromatischer Kohlenwasserstoffrest mit 6 bis 14 Kohlenstoffatomen, zum Beispiel ein Phenyl-, Naphthyl- oder Anthrylrest vorzugsweise ein Phenylrest, zu verstehen.

Den Alkylenteil der Aralkylgruppen bilden geradkettige, verzweigte, gesättigte oder ungesättigte Alkylenreste mit 1 mit 4 Kohlenstoffatomen wie Methylen, Ethylen, Vinylen, Propylen, Propenylen, Isopropylen, Butylen oder Ethylethylen.

Die gegebenenfalls an den Aryl- und Aralkylresten vorgesehenen Substituenten sind vorzugsweise $C_1$- bis $C_6$-Alkyl, $C_1$- bis $C_6$-Alkoxy, $C_1$-$C_6$-Alkylthio, Halogen, Hydroxyl, Formyl, Carboxyl, Amino, Nitro, Cyano, Aryloxy, Aryl, Aralkyl, wobei unter Halogen Brom, Chlor, Fluor oder Jod, vorzugsweise Chlor oder Fluor verstanden wird, Aryloxy zum Beispiel Phenoxy, Naphthoxy oder Anthryloxy, vorzugsweise Phenoxy darstellt, Aryl die oben angegebene Bedeutung hat und bevorzugt Phenyl ist und Aralkyl die oben angegebene Bedeutung hat und bevorzugt Benzyl bedeutet.

Unter einem alicyclischen Rest ist eine gegebenenfalls ungesättigte cycloaliphatische Gruppe mit 6 bis 10 Kohlenstoffatomen z. B. ein Cyclohexyl-, Cyclopentyl-, oder Cyclohexenylrest, vorzugsweise ein Cyclohexylrest zu verstehen.

Unter einem heterocyclischen Rest ist z. B. ein Pyridinyl-ein Piperidyl-, Morpholinyl- oder Tetrahydropyranylrest zu verstehen.

Die oben genannten Reste können soweit dort nichts anderes angegeben ist gegebenenfalls durch eine oder mehrere der folgenden Gruppen, wie z. B. Hydroxyl, Formyl, Carboxyl, Amino, Cyano oder Halogenatome substituiert sein.

Für die Verbindungen, die man aus Zwiebelpreßsaft isolieren kann, wurde gefunden, daß $R_1 = R_2 =$ Methyl oder Propyl; daß, wenn $R_1$ Methyl oder 1-Propenyl ist, $R_2$ 1-Propenyl oder Methyl ist, oder daß, wenn $R_1$ oder 1-Propenyl ist, $R_2$ 1-Propenyl oder Propyl ist.

Der Begriff Entzündung, wie er im Sinne der Erfindung zu verstehen ist, wird allgemein als Reaktion des Organismus und seiner Gewebe gegen verschiedenartige schädigende Reize definiert. Unter schädigenden Reizen sind exogene und auch endogene Reize, wie z. B. Gewebsverletzungen, das Eindringen von Fremdkörpern, chemische Stoffe, bakterielle Toxine, Allergene, Immunkomplexe, Mikroorganismen, krankhafte Stoffwechselprodukte sowie Zerfallsprodukte von Geschwülsten zu verstehen. Eng mit dem Entzündungsgeschehen verbunden sind die klassischen Symptome Schmerz und Fieber.

Es ist seit langem bekannt, daß bestimmte körpereigene Substanzen, die sogenannten Mediatoren, eng mit dem Entzündungsablauf verbunden sind. Diese Mediatoren, denen eine außerordentlich große pathogenetische Bedeutung zukommt, werden durch das schädigende Ereignis (Noxe) aus den Körperzellen freigesetzt. Als wichtigste und bekannteste Mediatoren gelten Histamin, 5-HT (5-Hydroxytryptamin), Bradykinin, die Prostaglandine, Prostacycline die Leukotriene, Thromboxane und der erst in neuerer Zeit besser erforschte Thrombozyten aktivierende Faktor (im folgenden als PAF bezeichnet; engl.: platelet activating factor).

Diese und andere Mediatoren wirken außerordentlich stark auf die Kontraktion der glatten Muskulatur, führen zu Herzfunktionsstörungen, beeinträchtigen die Integrität von Blutgefäßen und Schleimhäuten, wie z.B. die des Bronchialsystems; sie bewirken außerdem die Aggregation der Thrombozyten und polymorphkernigen Leukozyten mit den schwerwiegenden Folgen einer anaphylaktischen Atemwegsverengung, Blutdruckabfall, Herz-Arrythmien, Plasmaexudation, Gewebsödeme, Hämokonzentration, Thrombozytopenie, Leukozytopenie, Verklumpung der Thrombozyten und polymorphkernigen Leukozyten in den Lungenkapillaren, schwerste Atmungsstörungen und Kreislaufkollaps.

Aufgrund ihres breiten pharmakologischen Wirkungsspektrums, ihrer großen Verbreitung im Organismus, ihrer Bildung durch zahlreiche physikalische, chemische, pathologische, pathophysiologische und pharmakologische Einflüsse sowie aufgrund ihrer Beteiligung bei einer Vielzahl pathophysiologischer Abläufe sind die Mediatoren bzw. deren pharmakologische Beeinflussung von größter medizinischer Bedeutung (vgl. The Pharmacological Basis of Therapeutics, Ed. Goodman and Gilman, 6. Auflage, 1980, McMillan Publishing Company).

Bevorzugt wird erfindungsgemäß die Anwendung der Thiosulfinsäurederivate bei PAF induzierten pathologischen Zuständen weshalb im folgenden näher auf den Thrombozyten aktivierenden Faktor (PAF) eingegangen werden soll.

PAF ist ein Glycerophosphocholin mit der chemischen Bezeichnung 1-0-Alkyl-2-acetyl-sn-glyceril-3-phosphorylcholin. Es wird von einer Reihe von Zellen, wie z.B. Makrophagen, basophilen und nuetrophilen Granulozyten u.a. als Folge einer allergenbedingten Aktivierung dieser Zellen freigesetzt. Die Freisetzung von PAF bewirkt eine Reihe der bereits oben beschriebenen pathologischen Zustände, wobei PAF nicht direkt wirken muß, vielmehr seine Wirkung über eine Stimulierung weiterer Mediatoren entfalten kann. Neuere Untersuchungen zeigen insbesondere eine wichtige Rolle des PAF in der Genese des klinischen Bronchialasthmas sowie bei anderen pathologischen Zuständen der Lunge, z.B. der obstruktiven Bronchitits.

Neben seiner wichtigen Rolle bei der Genese des Bronchialasthmas und bei der Anaphylaxie ist PAF als hochpotenter Entzündungsmediator mit den bereits oben beschriebenen pathologischen Wirkungen anzusehen.

Eine Vielzahl der oben genannten Mediatoren, einschließlich des PAF, werden durch eine membrangebundene Phospholipase aus Phospholipiden der Zellmembran freigesetzt, wobei einerseits Arachidonsäure und andererseits eine PAF Vorstufe gebildet wird.

Ausgehend von Arachidonsäure werden zwei Gruppen von Mediatoren gebildet:

i) durch das Enzyme Cyclooxygenase die Prostaglandine einschließlich des Prostacyclins und des Thromboxans;

ii) durch das Enzym Lipoxygenase die offenkettigen Hydroperoxy- und Hydroxysäuren und insbesondere die Leukotriene.

Die PAF Vorstufe wird durch eine Acetyltransferase in die aktive Verbindung überfuhrt.

Pharmakologisch bedeutsam bei der Behandlung von Entzündungen sind zwei Gruppen von Wirksubstanzen; es handelt sich einmal um die sogenannten nicht-steroidalen Antiphlogistika, das sind Verbindungen und Derivate der Salicylsäure. Weitere Verbindungen mit bekannter antiphlogistischer Wirkung sind die Pyrazolonderivate, die para-Aminophenolderivate, die Indolderivate, (z.B. Indomethacin) und Derivate der Propionsäure. Die pharmakologische Wirkung aller dieser Verbindungen beruht darauf, daß sie die Cyclooxygenase zu hemmen vermögen und damit Synthese der Prostaglandine oder Thromboxane unterbinden.

Salicylsäure bzw. ihre Derivate und die weiteren Verbindungen der gesamten Klasse sind mit einer Reihe schwerer und schwerster Nebenwirkungen behaftet. So führt z.B. die längerdauernde Verabreichung von Salicylsäurederivaten zur Magen- und Darmulzeration. Ebenfalls bekannt sind die relative Unverträglichkeit der Pyrazolonderivate, die hepatotoxische Wirkung der para-Aminophenolderivate, die allgemeine Unverträglichkeit von Indomethacin sowie die ulzerative Wirkung der Propionsäurederivate.

Ein weiterer ebenfalls schwerwiegender Nachteil der nicht-steroidalen Antiphlogistika besteht darin, daß sie unter Umständen die pathologische Wirkung der Mediatoren verstärken, indem durch die Hemmung der Cyclooxygenase vermehrt Substrat für die Lipoxygenase und damit für die Bildung von Leukotrienen bereit gestellt wird.

Den nicht-steroidalen Antiphlogistika gegenüber stehen die steroidalen Antiphlogistika, das sind die Corticosteroide und ihre Derivate. Die antiphlogistische Wirkung der Corticosteroide beruht auf ihrer Fähigkeit, die Phospholipase als auch die Lipoxygenase zu hemmen wodurch der gesamte Arachidonsäurestoffwechsel inhibiert wird. Nachteilig für die Therapie mit Corticosteroiden sind die fatalen Nebenwirkungen, von denen nur beispielhaft die folgenden erwähnt werden sollen:
Ulcera duodeni oder ventriculi, Myopathie, Osteoporosen, pyschische Störungen, erhöhte Infektionsanfälligkeit, subkapsuläre Katarakte und dergleichen.

Neben den Corticosteroiden gibt es selektive Lipoxygenasehemmer, wie z.B. Benoxaprofen. Auch diese Substanzen sind mit schweren Nebenwirkungen behaftet, wie z.B. tödlich verlaufende exfoliative Dermatitiden (Syndrom der verbrühten Haut).

Die erfindungsgemäß verwendeten Verbindungen hemmen sowohl die Cyclooxygenase, die Thromboxansynthetase als auch die Lipoxygenase und besitzen darüberhinaus noch die Eigenschaft PAF induzierte Effekte zu blockieren. Sie besitzen daher die Eigenschaften der steroidalen Antiphlogistika, ohne jedoch deren fatale Nebenwirkungen aufzuweisen, und sind daher wertvoll zur Behandlung von Entzündungsvorgängen im weitesten Sinn, an deren Entstehung die bekannten Mediatoren wie z.B. Prostaglandine, Histamin, PAF, Leucotriene und Thromboxane eine Rolle spielen.

Im Gegensatz zu den nicht-steroidalen Antiphlogistika, die nicht gegen die von Leukozyten ausgelösten Aspekte von chronischen entzündlichen Erkrankungen wirken, da sie die Leukotrien-Bildung nicht inhibieren, sind die hier verwendeten Verbindungen aufgrund ihrer überraschenden, ausgezeichneten und vorteilhaften Wirkung auch in dieser Hinsicht den bekannten Verbindungen überlegen.

Entzündungshemmende Corticosteroide hemmen indirekt die Produktion sowohl der Prostaglandine als auch der Leukotriene, wodurch man zumindest zum Teil ihre ausgezeichneten therapeutischen Wirkungen erklärt. Wie oben beschrieben, ist die Behandlung mit Steroiden mit schwerwiegenden lokalen und auch systemischen Nebenwirkungen verbunden, so daß eine längere Verabreichung von Corticosteroiden bei vielen entzündlichen Zuständen kontraindiziert ist.

Die erfindungsgemäße Verwendung der Verbindungen erfüllt das Erfordernis für nicht-steroidale Antiphlogistika, die die Synthese der Leucotriene und Prostaglandine und Thromboxane inhibieren und den Effekten der PAF entgegenwirken. Sie sind frei von den mit den steroidalen oder nicht-steroidalen Antiphlogistike verbundenen Nachteilen.

Die Verbindungen sowie ihre pharmazeutisch annehmbaren Zubereitungen können daher in niedrigen Dosen zur Erzielung einer therapeutischen entzündungshemmenden Wirkung verabreicht werden.

Die Erfindung wird durch die im folgenden angegebenen Beispiele näher erläutert:
Bevorzugt im Sinne der vorliegenden Erfindung sind außer den in den Beispielen genannten Verbindungen die folgenden:

4-Brom-benzolthiosulfinsäure-S-phenylester
4-Chlor-benzolthiosulfinsäure-S-phenylester
3-Methyl-benzolthiosulfinsäure-S-phenylester
2,4-Dimethyl-benzolthiosulfinsäure-S-phenylester
4-Ethoxy-benzolthiosulfinsäure-S-phenylester
4-Chlor-benzolthiosulfinsäure-S-4-methoxy-phenylester
3-Nitro-benzolthiosulfinsäure-S-4-methoxy-phenylester
4-Methylthio-benzolthiosulfinsäure-S-phenylester
4-Phenyl-benzolthiosulfinsäure-S-phenylester

Benzolthiosulfinsäure-S-2-carboxy-phenylester
2-Phenoxy-benzolthiosulfinsäure-S-phenylester
Pyridin-3-thiosulfinsäure-S-phenylester
Benzolthiosulfinsäure-S-(2-pyridyl) ester
Benzolthiosulfinsäure-S-(2-naphthyl) ester
Naphthalin-2-thiosulfinsäure-S-phenylester
Benzolsulfinsäure-S-2-amino-phenylester
Benzolthiosulfinsäure-S-4-hydroxy-phenylester
Benzolthiosulfinsäure-S-2,6-dichlor-phenylester
Benzolthiosulfinsäure-S-3,5-di-t-butyl-4-hydroxy-phenylester.
Die Verbindungen zur Verwendung im Sinne der vorliegenden Erfindung können zum Beispiel durch das folgende Verfahren aus Zwiebeln isoliert werden:

Beispiel 1

A) Ausgangsmaterial:

Man gewinnt aus geschälten, zerkleinerten und homogenisierten Zwiebeln (Allium cepa) Zwiebelpreßsaft. Das Homogenat wird vor dem Pressen mindestens 20 min stehengelassen. Der Preßsaft wird zweimal mit Dichlormethan, Chloroform oder anderen mit Wasser nicht mischbaren organischen Lösungsmitteln ausgeschüttelt; die organische Phase wird über Natriumsulfat getrocknet, abfiltriert und das Lösungsmittel unter vermindertem Druck abdestilliert. Man erhält einen gelblich-grünen öligen Rückstand.
Dieser Rückstand wird durch chromatographische Verfahren fraktioniert.

B) Fraktionierung/Methode I:

1. Zur Grobfraktionierung eignet sich z. B. die Säulenchromatographie an Kieselgel mit Toluol/Ethylacetat (10 : 3) als Eluens.
2. Anschließend liefert eine erneute säulenchromatographische Auftrennung angereicherter Fraktionen der aktiven Substanzen an Kieselgel mit z.B. Dichlormethan/Aceton (100 : 1) als Eluens die erfindungsgemäßen Verbindungen.

Für diesen Trennungsschritt sind Laufmittelsysteme auf der Basis von Chloroform oder Dichlormethan mit Beimenungen von 1 bis 5 % Aceton, Ethylmethylketon, Diethylether oder Laufmittelsysteme auf der Basis von Kohlenwasserstoffen wie Heptan, Pentan oder Hexan mit Beimenungen von 5 bis 10 % Aceton oder Ethyl-Methylketon geeignet.

Beispiel 1a

B) Fraktionierung/Methode II:

Die Isolierung der Verbindungen kann ausgehend vom Rückstand der organischen Ausschüttelung aus Zwiebelpreßsaft wie folgt vereinfacht werden:
1. Die sich auf Kieselgel zum Teil mit den zu isolierenden Substanzen überlagernden Triterpene werden zunächst durch "Flash-Chromatographie" an reversed-phase Material (z. B. RP 8 oder RP 18) unter Verwendung eines hydrophilen Laufmittels (z. B. Methanol) abgetrennt.
2. Danach ist mit Hilfe der Mitteldruckflüssigkeitschromatographie (MPLC) die Isolierung der Thiosulfinsäureester in einem Schritt möglich. Als stationäre Phase dient wiederum Kieselgel, als Eluens die Lösungsmittelgemische des zweiten Fraktionierungsschrittes aus Methode I. (siehe Beispiel 1)

Beispiel 2

Aufgrund der guten Löslichkeit der Verbindungen in niederen Alkoholen (z. B. Methanol, Ethanol) ist auch eine Alkoholextraktion aus z. B. lyophilisiertem Zwiebelhomogenat möglich. Im Anschluß kann nach Methode I (Beispiel 1) oder II (Beispiel 1a) fraktioniert werden.

Beispiel 3

Extraktion:

Der Zwiebelpreßsaft wird mit organischen Lösungsmitteln ausgeschüttelt, die mit Wasser nicht mischbar sind, wie z. B. : Dichlormethan, Chloroform, Diethylether und weitere Lösungsmittel vergleichbarer Polarität. Organische Lösungsmittel sehr geringer Polarität wie z. B. Petrolether sind nicht geeignet.

Beispiel 4

Der Saft oder auch das Homogenat können auch mit superkritischen Gasen wie z. B. $CO_2$ unter Druck extrahiert werden.

Man erhält nach jedem der vorstehenden Beispiele jeweils sechs Thiosulfinsäurederivate mit den folgenden Formeln:

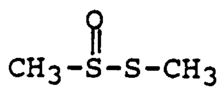 (II)

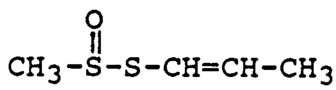 (III)

(trans)

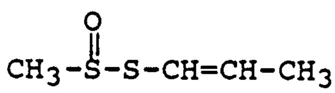 (IV)

(cis)

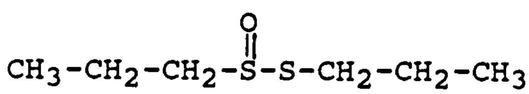 (V)

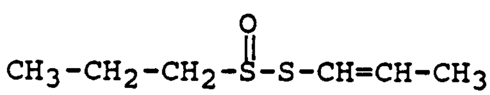 (VI)

(cis)

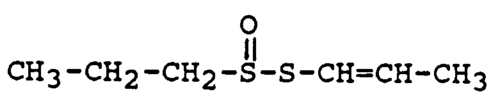 (VII)

(trans)

Die Thiosulfinsäurederivate der Formeln (III) bis (VII) sind neue Produkte

Die Strukturen der Verbindungen VI und VII sind unter Umständen vertauscht zuzuordnen.

Die Lagerung der Extrakte, Fraktionen und Reinsubstanzen erfolgt unter Ausschluß von Licht und Sauerstoff bei - 20 °C.

Chromatographische Charakterisierung der aus Zwiebeln isolierten Thiosulfinsäureester

1) Dünnschichtchromatographie:

a) Stationäre Phase: HPTLC-Fertigplatten Kieselgel 60F 254, Schichtdicke 0,25 mm
(b) Mobile Phase

| | Verbindung | $R_f$-Wert |
|---|---|---|
| Toluol/Ethylacetat (10:3) | II | 0,25 |
| | III | 0,31 |
| | IV | 0,35 |
| | V | 0,44 |
| | VI | 0,46 |
| | VII | 0,50 |
| Chloroform/Aceton (100:4) | II | 0,47 |
| | III | 0,49 |
| | IV | 0,49 |
| | V | 0,54 |
| | VI | 0,57 |
| | VII | 0,57 |
| Dichlormethan/Aceton (100:1) | II | 0,28 |
| | III | 0,34 |
| | IV | 0,38 |
| | V | 0,37 |
| | VI | 0,40 |
| | VII | 0,44 |

c) UV-Verhalten:
Alle Thiosulfinsäureester zeigen im UV 254 eine deutliche Fluoreszenzminderung.
(d) Detectionsverhalten:

| Sprühreagens | Verbindung | Reaktion |
|---|---|---|
| - Ethanolische Schwefelsäure 5%/Vanillin (1% in EtOH)/110 °C | II<br><br>III<br>IV<br>V<br>VI<br>VII | keine<br><br>schwach braunrot<br>dto<br>keine<br>schwach rötlich<br>dto |
| - Palladium(II)-chloridlösung 1 % in Wasser | II<br><br>III<br>IV<br>V<br>VI<br>VII | gelbbraun<br><br>braun<br>braun<br>hellgelb<br>braun<br>braun |
| - 5 % Natrium-Nitroprussitnatrium in Wasser/Ethanolische Natronlauge (10 %) | II<br><br>III<br>IV<br>V<br>VI<br>VII | hellrot<br><br>dto<br>dto<br>dto<br>dto<br>dto |

2) Hochdruckflüssigkeitschromatographie:

a) Stationäre Phase: Hibar Fertigsäule RT 125-4; Lichrospher 100 CH-18/2, 5 um
b) Mobile Phase: Acetonitril/Wasser-Gradientensystem
c) Detektion bei 200 nm
d) Retentionszeiten:
Bei Verwendung eines Gradientensystems von 20 % auf 80 % Acetonitril in 30 min ergeben sich Retentionszeiten von 1,80 min (Verbindung II), 4,25 min (Verbindung III), 4,40 min (Verbindung IV), 10,19 min (Verbindung V), 9,97 min (Verbindung VI) und 10,19 min (Verbindung VII).

Weiterhin können die Verbindungen auf chemischem Weg nach an sich bekannten Verfahren zur Herstellung analoger Verbindungen hergestellt werden:

Beispiel 5

Benzolthiosulfinsäure-S-phenylester

Zu einer Lösung von 2.2 g (20 mmol) Thiophenol in 30 ml trockenem Ether gibt man 1.8 g Pyridin und tropft hierzu unter Kühlung eine Lösung von 3.2 g (20 mmol) Benzolsulfinsäurechlorid in 20 ml trockenem Ether. Man läßt 10 min nachrühren, gießt in verd. Schwefelsäure, trennt die organische Phase ab, trocknet und engt ein. Es verbleiben 3.2 g Titelverbindung (68 % d. Th.) vom Schmp. 69-70 °C (aus Ligroin).

Beispiel 6

In analoger Weise wie in Beispiel 5 beschrieben erhält man:

| | Bezeichnung | Ausb. % | Schmelzpunkt °C |
|---|---|---|---|
| a) | Benzolthiosulfinsäure-S-4-methyl-phenylester | 81 | 68 |
| b) | 4-Methyl-benzolthiosulfinsäure-S-phenylester | 71 | 70 -71 |
| c) | 4-Methyl-benzolthiosulfinsäure-S-4-methyl-phenylester | 86 | 86 |
| d) | Benzolthiosulfinsäure-S-4-t-butyl-phenylester | 81 | 70 |
| e) | 4-t-Butyl-benzolthiosulfinsäure-S-phenylester | 81 | 80 -81 |
| f) | 4-t-Butyl-benzolthiosulfinsäure-S-4-t-butyl-phenylester | 74 | 104 - 105 |
| g) | Benzolthiosulfinsäure-S-4-methyoxy-phenylester | 68 | 75 -77 |
| h) | 4-Methoxy-benzolthiosulfinsäure-S-phenylester | 72 | 61 -62 |
| i) | 4-Methoxy-benzolthiosulfinsäure-S-4-methoxy-phenylester | 91 | 90 -91 |
| j) | 4-Methyl-benzolthiosulfinsäure-S-4-methoxy-phenylester | 75 | 79 |
| k) | 4-Methoxy-benzolthiosulfinsäure-S-4-methyl-phenylester | 90 | 83 |
| l) | 4-Methyl-benzolthiosulfinsäure-S-4-chlor-phenylester | 77 | 92 -93 |

Beispiel 7

Man erhält Thiosulfinsäurederivate, in denen $R_1$ und $R_2$ die gleiche Bedeutung haben in Analogie zu Kametani et al, Jap. J. Pharm. Chem. 31 (1959), S. 60 und Small, Bailey et al J. Am. Chem. Soc - 69, (1947), S. 1712 durch Oxidation der entsprechenden Disulfide mit einer äquimolaren Menge 3-Chlorperbenzoesäure (bzw. Perbenzoesäure oder Peressigsäure) bei O°C in Dichlormethan (bzw. Trichlormethan oder Essigsäure).

0,05 mol Dipropylsulfid werden in 300 ml Dichlormethan gelöst und die Lösung im Eisbad gekühlt. Mit Hilfe eines Scheidetrichters werden 0,045 mol 3-Chlorperbenzoesäure gelöst in 75 ml Dichlormethan unter Rühren langsam zugetropft. Danach wird weitere 15 min gerührt und der Niederschlag abfiltriert. Nach dem Waschen mit je 300 ml 5 %iger $NaHCO_3$-Lösung, 3 %iger $NaHCO_3$-Lösung und Aqua dem. wird die organische Phase über $Na_2SO_4$ getrocknet und das Lösungsmittel unter vermindertem Druck abgezogen.

Die Reinigung von Nebenprodukten und nicht umgesetzten Dipropylsulfid erfolgte mittels Chromatotron oder Flash-Chromatographie an Kieselgel: Dabei wird zunächst nicht umgesetztes Dipropysulfid mit n-Hexan eluiert, danach Propylthiosulfinsäurepropylester und Propylthiosulfonsäurepropylester mit Dichlormethan:Aceton (100 : 1) getrennt. Die Identifikation der öligen Substanz erfolgt durch Ultraviolet-Spektroskopie ( $_{max}$ = 240 nm (Diodenarray) und HPLC-Chromatographie Rt: 9,9 min.

Für die Hochdruckflüssigchromatographie (HPLC) wird reversed phase Material mit Acetonitril/Wasser Gradientenelution verwandt. Um auch Substanzen ohne chromophore Gruppen zu erfassen, wird bei 200 nm detektiert.

In einem Universalsystem für alle bearbeiteten schwefelhaltigen Verbindungen steigt die Acetonitrilkonzentration in 30 min linear von 20 % auf 80 %.

Die Ausbeute beträgt 65 % d. Th. .

Beispiel 8

In analoger Weise wie in Beispiel 7 werden erhalten

a) aus Dimethyldisulfid der Methylthiosulfinsäuremethylester.
Ausbeute: 50 % d. Th.
Schmp.: farbloses, leicht bewegliches Öl
Rt: 1,7 min (HPLC)
UV: $_{max}$ = 240 nm (Diodenarray)

(Die Abtrennung des Thiosulfinsäure-Ester von Thiosulfonsäure-Ester erfolgt im Laufmittelsystem Dichlormethan/Acetan (100:2));
b) aus Diallydisulfid der Allylthiosulfinsäureallylester
Ausbeute: 10 % d. Th.
Schmp.: Öl
Rt: 12,3 min (HPLC)
UV: $_{max}$ = 260 nm (Diodenarray)
Laufmittelsystem: Dichlormethan/Aceton (100:1) ;
und
c) aus Diphenyldisulfid der Phenylthiosulfinsäurephenylester Ausbeute: 75 % d. Th.
Schmp.: 62 - 63 °C
Rt: 18,4 min (HPLC)
UV: $_{max}$ = 222, 283 nm (Diodenarray)
Laufmittelsystem: Dichlormethan/n-Hexan (1:1).

Beispiel 9

Methylthiosulfinsäurephenylester und Phenylthiosulfinsäuremethylester

0,2 mol Thiophenol werden zusammen mit 0,2 mol Dimethyldisulfid unter Zusatz von 80 ml 1 %iger methanolischer KOH und 10 ml Wasser in 500 ml Methanol gelöst.
Der Rekationsansatz wird 3 h im Abzug; unter Rückfluß zum Sieden erhitzt, bis die HPLC-Untersuchung die Abwesenheit von Dimethyldisulfid anzeigt.
Anschließend wird die methanolische Lösung mit 300 ml Wasser versetzt und 3 x mit 500 ml n-Pentan ausgeschüttelt. Die Pentanphase wird getrocknet und das Lösungsmittel abdestilliert.
Die erhaltene Disulfide werden dann wie in Beispiel 7 oxidiert und der Ansatz entsprechend aufgearbeitet.
Die Gewinnung der reinen Thiosulfinate erfolgt durch Mitteldruck-Flüssigchromatographie (MPLC) an Kieselgel mit Dichlormethan als Elutionsmittel.

I)Methylthiosulfinsäurephenylester

Öl
Rt: 7,8 min (HPLC) UV: $_{max}$ = 223 (sh), 250 nm (Diodenarray)

II)Phenylthiosulfinsäuremethylester

Öl
Rt: 9,2 min (HPLC)
UV: $_{max}$ = 220 (sh) , 260 nm (Diodenarray)

Beispiel 10

n-Propylthiosulfinsäuremethylester und Methylthiosulfinsäure-n-propylester

In analoger Weise wie in Beispiel 9 werden aus Propanthiol und Dimethyldisulfid die beiden Titelverbindungen hergestellt.

Beispiel 11

Die aus Allium cepa L. isolierten Thiosulfinate sind auch durch Inkubation von synthetisierten (+)-Alk(en)yl-L-cysteinsulfoxiden mit einer Alliinasepräparation unter geeigneten Bedingungen zugänglich.

Beispiel 12

Schließlich lassen sich die Verbindungen auch ausgehend von Sulfinsäurechloriden und Thiolen, wie von T.L. Moore, D.E. O'Connor für analoge Verbindungen beschrieben (J. Org. Chemistry, 31 (1966), S.

3587, herstellen.

Erfindungsgemäß können die Verbindungen zur Behandlung von Entzündungen der Gelenke, der Haut, der Schleimhäute sowie innerer Organe verwendet werden, unabhängig davon, ob dies durch Infektionserreger, immunologische Vorgänge oder Traumen hervorgerufen worden sind. Hierbei sind insbesondere Entzündungsvorgänge des Bronchialsystems, wie Asthma bronchiale oder obstruktive Bronchitis besonders vorteilhaft zu behandelnde Indikationen. Weiterhin können die Verbindungen zur Prophylaxe und Behandlung von Gefäß- und Herzerkrankungen verwendet werden, bei denen es wünschenswert erscheint, die Biosynthese von Entzündungsstoffen durch Thrombozyten zu behindern. Besonders vorteilhaft können die Verbindungen zur Prophylaxe oder Behandlung aller PAF und/oder Leukotrien induzierten Phänomene und insbesondere zur Behandlung des Bronchialraums verwendet werden.

Weiterhin wird gemäß der vorliegenden Erfindung eine Verbindung der allgemeinen Formel (I) zur Verwendung in einem Verfahren zur Inhibierung der Lipoxygenase, Cyclooxygenase und Thromboxansynthetase in einem Säugetier, beispielsweise dem Menschen, zur Verfügung gestellt. Die Erfindung stellt weiterhin die Verwendung einer Verbindung in der Behandlung oder Prophylaxe eines Säugetiers, einschließlich des Menschen, insbesondere zur Behandlung einer der vorhin beschriebenen Krankheiten oder Zuständen, zur Verfügung.

Die erforderliche Menge der zu verwendenden Verbindung (im folgenden als der aktive Bestandteil bezeichnet) für die therapeutische Wirkung hängt von der jeweiligen Verbindung, der Verabreichungsart und von dem zu behandelnden Subjekt, sowie der jeweiligen Krankheit ab. Eine geeignete Dosis einer Verbindung zur Verabreichung an ein Säugetier, das an einer Entzündung, einem schmerzhaften oder fiebrigen Zustand, wie vorhin beschrieben, leidet, beträgt etwa 0,1 $\mu$g bis 500 mg des aktiven Bestandteils pro Kilogramm Körpergewicht. Im Falle einer systemischen Verabreichung kann die Dosis im Bereich von 0,5 bis 500 mg der aktiven Verbindung pro Kilogramm Körpergewicht, und die am meisten bevorzugte Dosis im Bereich von 0,5 bis 50 mg pro Kilogramm Körpergewicht, zum Beispiel 5 bis 25 mg pro Kilogramm Körpergewicht, betragen, die zwei- oder dreimal täglich verabreicht wird.

Im Falle einer topischen Verabreichung, z. B. auf die Haut oder Schleimhäute, kann die geeignete Dosis deutlich höher liegen.

Obwohl es möglich ist, den aktiven Bestandteil alleine zu verabreichen, ist es vorzuziehen, daß dieser in Form einer pharmazeutischen Formulierung, die eine Verbindung gemäß der allgemeinen Formel und einen dafür pharmazeutisch annehmbaren Trägerstoff enthält, verabreicht wird. Üblicherweise liegt der aktive Bestandteil in einer derartigen Formulierung in einer Konzentration von 0,1 bis 99,9 Gew.-% der Formulierung vor. Üblicherweise enthält eine Einzeldosis einer Formulierung zwischen 0,1 mg und 1 g des aktiven Bestandteils. Für die topische Verabreichung beträgt die Konzentration des aktiven Bestandteils vorzugsweise 1 bis 2 Gew.-% der Zubereitung, jedoch kann der aktive Bestandteil bis zu 10 Gew.-% ausmachen. Zubereitungen, die für eine nasale oder buccale Verabreichung geeignet sind, wie z. B. selbstzerstäubende Pulver, können 0,1 bis 20 Gew.-%, z. B. 2 Gew.-%, des aktiven Bestandteils enthalten.

Die Zubereitungen für die erfindungsgemäße Verwendung in der Veterinär-als auch in der Humanmedizin enthalten den aktiven Bestandteil in Verbindung mit einem dafür pharmazeutisch annehmbaren Trägerstoff und gegebenenfalls weiteren therapeutisch aktiven Bestandteilen. Der Trägerstoff muß annehmbar in dem Sinne sein, daß er mit den anderen Bestandteilen der Formulierung kompatibel ist und keine nachteilige Wirkung auf den Empfänger der Formulierung besitzt.

Die Zubereitungen liegen geeigneterweise in Form einer oralen, ophthalmologschen, rektalen, parenteralen (einschließlich subkutanen, intraumuskulären und intravenösen), intraartikulären, topischen, nasalen oder buccalen Verabreichungsform vor.

Die Formulierungen liegen üblicherweise in Form einer Einzeldosis vor und können durch jedes der auf dem Gebiet der pharmazeutischen Technologie bekannten Verfahren hergestellt werden. Alle Verfahren enthalten den Schritt des Zusammenbringens des aktiven Bestandteils mit dem Trägerstoff, gegebenenfalls mit einem oder mehreren zusätzlichen Bestandteilen. Im allgemeinen werden die Zubereitungen durch gleichmäßiges und inniges Vermischen des aktiven Bestandteils mit einem flüssigen Träger oder einem fein verteilten festen Träger oder beiden und anschließend, falls erforderlich, Formen des Produktes in die erwünschte Zubereitungsform, hergestellt.

Die erfindungsgemäßen Zubereitungen zur oralen Verabreichung können in Form von diskreten Einheiten, wie zum Beispiel Kapseln, Cachets, Tabletten oder Pastillen, vorliegen, wobei jede Form eine vorbestimmte Menge des aktiven Bestandteils enthält. Sie können ebenfalls in Form eines Pulvers oder in Form von Granula oder in Form einer Lösung oder einer Suspension in einer wäßrigen oder nicht-wäßrigen Flüssigkeit, oder in Form einer Öl-in-Wasser-Emulsion oder Wasser-in-Öl-Emulsion, vorliegen. Der aktive Bestandteil kann ebenfalls in Form eines Bolus, eines Elektuariums oder einer Paste vorliegen.

Eine Tablette, die die Verbindung enthält, kann man durch Verpressen oder Vergießen des aktiven Bestandteils gegebenenfalls zusammen mit einem oder mehreren zusätzlichen Bestandteilen herstellen. Verpreßte Tabletten kann man durch Pressen des aktiven Bestandteils in freifließender Form, z. B. als Pulver oder als Granula, gegebenenfalls vermischt mit einem Bindemittel, einem Gleitmittel, einem inerten Verdünnungsmittel, oberflächenaktiven oder einem Dispersions-Mittel, in einer geeigneten Maschine herstellen. Gegossene Tabletten kann man durch Vergießen einer Mischung des in Pulverform vorliegenden aktiven Bestandteils und eines geeigneten Trägerstoffs, der mit einem inerten flüssigen Verdünnungsmittel befeuchtet ist, in einer geeigneten Maschine herstellen.

Die Formulierungen für die rektale Verabreichung in Form von Suppositorien vorliegen, wobei der aktive Bestandteil in einem Träger, z. B. aus Kakaobutter, enthalten ist, sie können auch in Form eines Klistiers vorliegen.

Die für die parenterale Verabreichung geeigneten Zubereitungen enthalten üblicherweise eine sterile wäßrige Zubereitung des aktiven Bestandteils, die vorzugsweise mit dem Blut des Empfängers isotonisch ist.

Zubereitungen, die für die intraartikuläre Verabreichung geeignet sind, können in Form einer sterilen wäßrigen Zubereitung des aktiven Bestandteils vorliegen, wobei der aktive Bestandteil gegebenenfalls in mikrokristalliner Form vorliegt, z. B. in Form einer wäßrigen mikrokristallinen Suspension. In gleicher Weise können liposomale Zubereitungen oder bioabbaubare Polymersysteme zur Verabreichung des aktiven Bestandteils benutzt werden.

Für die topische Verabreichung geeignete Zubereitungen enthalten flüssige oder halbflüssige Zubereitungen, wie z. B. Einreibemittel, Lotionen, Verbände., Öl-in-Wasser- oder Wasser-in-Öl-Emulsionen, wie z. B. Cremes, Salben oder Pasten, oder Lösungen oder Suspensionen, wie z. B. Tropfen. Zum Beispiel kann der aktive Bestandteil für die ophthalmologische Verabreichung in Form von wäßrigen Augentropfen, beispielsweise in Form einer 0,1 bis 1,0 %igen Lösung, vorliegen.

Für die Verabreichung durch die Nase oder in die Mundhöhle geeignete Zubereitungen liegen in Form eines selbstzerstäubenden Pulvers oder in Form von Sprayzubereitungen, wie z. B. Aerosole, vor. Die Zubereitungen ergeben nach Dispersion vorzugsweise eine Teilchengröße im Bereich von 10 bis 200 $\mu$m.

Zubereitungen die gemäß der vorliegenden Erfindung verwendet werden, können den aktiven Bestandteil auch in einer wäßrigen oder verdünnten alkoholischen Lösung enthalten. Der aktive Bestandteil kann gegebenenfalls durch ein Sprühgerät in einen feinen Nebel überführt werden, der von Patienten inhaliert wird. Derartige Zubereitungen enthalten üblicherweise ein Geschmacksmittel, wie z. B. Natriumsaccharin und ein ätherisches Öl. Ebenfalls kann eine Puffersubstanz und/oder ein overflächenaktives Mittel in derartigen Zubereitungen zusammen mit Konservierungsmitteln, wie z. B. Methylhydroxybenzoat enthalten sein.

Andere Zubereitungen, die zur Verabreichung durch die Nase geeignet sind, bestehen aus einem groben Pulver, das eine Teilchengröße von 20 bis 500 $\mu$m aufweist, das auf die gleiche Art und Weise wie Schnupftabak verabreicht wird.

Zusätzlich zu den vorhin genannten Bestandteilen können die Zubereitungen zur erfindungsgemäßen Verwendung einen oder mehrere zusätzliche Bestandteile wie z.B. Verdünnungsmittel, Puffersubstanzen, Geschmacksstoffe, Bindemittel, oberflächenaktive Mittel, Verdickungsmittel, Gleitmittel, Konservierungsmittel, z.B. Methylhydroxybenzoat (einschließlich Antioxidantien), Emulgiermittel und dergleichen enthalten.

Anschließend wird die Wirkung der Verbindungen der allgemeinen Formel (I) anhand von in vitro- und in vivo-Versuchen gezeigt.

1. Untersuchung der asthmaprotektiven Wirkung der Verbindufgen in vivo. Eingesetzt wurde eine Mischung der Verbindungen 1) (identisch mit Formel III von S. 13) und Verbindung 2) (identisch mit Formel IV von S. 13) Das Verhältnis von 1) : 2) betrug ca. 1 : 2.

Zur Untersuchung der asthmaprotektiven Wirkung in vivo wurden entsprechende Versuche mit Meerschweinchen durchgeführt (Dorsch W. et. al., Pflügers Arch. 391, Seite 263, (1981)).

a) Protektive Wirkung bei PAF induziert Bronchialobstruktion.

7 Meerschweinchen wurden entsprechend einem randomisiertem Überkreuzprotokoll entweder mit dem aktiven Bestandteil in Olivenöl gefüttert oder sie erhielten den Trägerstoff Olivenöl allein. Die Dosis des aktiven Bestandteils betrug 20 mg pro kg Körpergewicht. 30 min später inhalierten die Tiere 10 $\mu$g PAF. Die resultierende Bronchialobstruktion wurde ganzkörperplethysmographisch in 5 min Abständen für die Dauer von 30 min erfaßt (als Maß der Bronchialobstruktion wird der Parameter "compressed air" benutzt).

Die Ergebnisse sind in der folgenden Tabelle 1 sowie in der Abbildung 1 dargestellt.

Tabelle I

Asthmatische Reaktionen von Meerschweinchen (0,1 ml compressed air) auf die Inhalation von PAF

| Tier | Kontrolle | | | | | | | Verb. (20 mg/kg oral) | | | | | | |
|---|---|---|---|---|---|---|---|---|---|---|---|---|---|---|
| | 0' | 5' | 10' | 15' | 20' | 25' | 30' | 0' | 5' | 10' | 15' | 20' | 25' | 30' |
| 1 | 0 | 5 | 5 | 3 | 3 | 3 | 3 | 0 | 4 | 2 | 0 | 1 | 1 | 1 |
| 3 | 0 | 6 | 8 | 6 | 3 | 3 | 3 | 0 | 3 | 3 | 1 | 1 | 1 | 1 |
| 4 | 0 | 2 | 7 | 6 | 4 | 4 | 4 | 0 | 5 | 3 | 2 | 0 | 1 | 1 |
| 5 | 0 | 6 | 5 | 3 | 1 | 1 | 2 | 0 | 4 | 4 | 4 | 3 | 3 | 3 |
| | (Wiederbelebung nötig) | | | | | | | | | | | | | |
| 6 | 0 | 7 | 6 | 4 | 4 | 4 | 2 | 0 | 6 | 5 | 3 | 0 | 1 | 0 |
| 7 | 0 | 12 | 5 | 2 | 3 | 2 | 1 | 0 | 3 | 4 | 3 | 3 | 3 | 2 |
| 8 | 0 | 6 | 6 | 6 | 3 | 5 | 3 | 0 | 6 | 2 | 2 | 2 | 1 | 1 |
| | (Wiederbelebung nötig) | | | | | | | | | | | | | |

In weiteren Versuchen wurden anstelle der Mischung aus Verbindung 1) und 2) Verbindungen eingesetzt, worin $R_1$ und $R_2$ $CH_3$ (Verbindung 3, vgl. Bsp. 8a) bzw. worin $R_1$ und $R_2$ Phenyl (Verbindung 4, vgl. Bsp. 8c) ist. Die Ergebnisse sind in Abb. 2 bzw. Abb. 3 dargestellt. Im Falle der Verbindung 3) betrug die Dosis 50 mg/kg und im Falle der Verbindung 4) 100 mg/kg.

Aus der Tabelle sowie aus den Abb. 1 bis 3 kann man unschwer entnehmen, daß bei den mit den Verbindungen behandelten Tieren die Wirkung des verabreichten PAF wesentlich schwächer ist als bei den Kontrolltieren (größere Zahlen bedeuten schweres Asthma). Der Unterschied war statistisch signifikant: Abb. 1; 5 min nach Inhalation $p < 0,02$, 10 min. $p < 0,001$ (t-Test nach Student für gepaarte Daten).

b) Protektive Wirkung beim allergeninduzierten Asthma bronchiale von Meerschweinchen

9 Meerschweinchen wurden - wie unter 1a) beschrieben - entsprechend einem randomisierten Überkreuzprotokoll entweder mit dem aktiven Bestandteil in Olivenöl gefüttert oder dem Träger Olivenöl allein. Die Dosis der verabreichten Verbindung betrug 20 mg/kg Körpergewicht 30 Minuten später inhalierten die Tiere zweimal im Abstand von 10 Minuten jeweils 20 Sekunden lang eine einprozentige Ovalbuminlösung, die Substanz, gegenüber der sie 3 Wochen vorher sensibilisiert worden waren. Das Ausmaß der resultierenden Bronchialobstruktion wurde ebenfalls ganzkörperplethysmographisch in 2-minütigen Abständen für die Dauer von zweimal 10 Minuten erfaßt (zur Methode siehe: Dorsch W., Walter O., Rosmanith J.: Continuous Recording of Intrapulmonary "Compressed Air" as a Sensitive Noninvasive Method of Measuring Bronchial Obstruction in Guinea Pigs. Pflügers Arch. 391: 236-241 (1981)).

Das Ergebnis dieser Versuche ist in Abbildung 4 für die Mischung der Verbindung 1) und 2) und in den Abbildungen 5 und für die Verbindung 3) bzw. 4) dargestellt. Verabreichte Dosis wie in 1a). Aus den Abbildungen geht hervor, daß die verabreichten Substanzen die asthmatische Reaktion von Meerschweinchen auf die Inhalation eines Allergens deutlich abschwächen ($p<0,05$, $p<0,05$ beim Vergleich der Maxima u. Minima der asthmatischen Reaktion; Abb. 4; t-Test nach Student für gepaarte Daten).

2. Untersuchung der enzymhemmenden Wirkung der Verbindungen in vitro.

Die enzymhemmende Wirkung der Verbindungen wurden in vitro bestimmt; Verwendung der Mischung aus Verbindung 1) und 2).

a) Inhibition der Thromboxanbiosynthese von thrombinstimulierten plättchenreichen Plasma.

Von einem menschlichen Spender wurde-plättchenreiches Plasma in der üblichen Methodik gewonnen und auf eine Plättchenzahl von 90.000/ml eingestellt. Dieses Plasma wurde entweder mit einem Lösungsmittel (1 % -Dimethylsulfoxyd (DMSO) - physiologische Kochsalzlösung) oder mit verschiedenen Konzentrationen der Verbindung inkubiert (s. Tabelle II).

Tabelle II

| | | Konzentration der Verbindungen (mg/ml) | | | | |
|---|---|---|---|---|---|---|
| | | 0,0 | 0,001 | 0,01 | 0,1 | 1,0 |
| | | Thromboxan $B_2$-Gehalt im Überstand von plättchenreichem Plasma (pg/ml) | | | | |
| Gesamtmenge an Thrombin (IU) | 0 | 280 | | | | |
| | 1 | 2 400 | 3 400 | 3 300 | 1 200 | 618 |
| | 10 | 15 900 | 14 500 | 8 600 | 2 500 | 828 |

Tabelle II: Einfluß der Verbindung auf die Biosynthese von Thromboxan $B_2$ in menschlichen Blutplättchen (pg/ml plättchenreiches Plasma = pg/90 000 Plättchen).

5 Minuten später wurden die Plättchen mit Thrombin (1 bzw. 10 Internationale Einheiten = I.U.) bei 37 °C stimuliert. Nach 15 Minuten wurde die Reaktion mit eiskaltem Methanol (Volumenverhältnis 1 : 1) gestoppt, danach durch 10-minütiges Zentrifugieren bei 3 000 U/min. (4 °C) getrennt. Im Überstand wurde nach üblicher Methodik radioimmunologisch Thromboxan $B_2$ bestimmt; das Ergebnis dieser Untersuchung zeigt Tabelle II. Aus ihr geht hervor, daß die Verbindungen die Biosynthese von Thromboxan in Thrombin-stimulierten menschlichen Blutplättchen konzentrationsabhängig hemmen.
Bei Einsatz von 0,001 mg/ml bis 1,0 mg/ml Vergbindung 3) bzw. Verbindung 4) findet man eine dosisabhängige Unterdrückung der Thromboxan $B_2$ Biosynthese von thrombinstumuliertem plättchenreichem Plasma von maximal 61% (Verbindung 3) bzw. 76% (Verbindung 4).

b) Hemmung der 5-Lipoxygenase-Aktivität von isolierten Schweineleukozyten Verwendung der Mischung aus Verbindung 1) und 2).

Schweineleukozyten entsprechen in ihrer biochemischen Aktivität menschlichen Leukozyten, d.h. aus den geschilderten Untersuchungsergebnissen lassen sich direkte Schlüsse auf die inhibitorische Aktivität der Verbindungen auf menschliche Granulozyten ziehen. Das Enzym 5-Lipoxygenase synthetisiert aus Arachidonsäure hochpotente Entzündungsstoffe, nämlich die Leukotriene $LTB_4$, $LTC_4$ und $LTD_4$.
Vollblut von Schweinen wurde mittels Heparin und Na-Citrat ungerinnbar gemacht, danach durch Dextransedimentation die Leukozyten isoliert. Nach Lyse verunreinigender Erythrozyten durch hypertone Ammoniumchloridlösung und mehrmaligem Waschen wurde die Zellzahl auf 4 x $10^7$ Zellen pro ml Phosphat-Puffer (ph 7,4) eingestellt. Durch Zugabe von 10 µmol Eikosatetraynsäure (ETYA) wurde selektiv die 12-Lipoxygenase blockiert. Die zu untersuchenden Verbindungen wurden in maximal 2 % äthanolischer Lösung gelöst und in folgenden Konzentrationen zu den Zellsuspensionen gegeben: 5, 10, 20 und 30 µmol.
Unmittelbar danach wurden die Zellen durch Zugabe von Calcium Ionophor (A 23187, 15 µmol) und radioaktiv markierter Arachidonsäure ($^{14}$C-Arachidonsäure, 0,7 µmol = 0,1 µCi) 5 Minuten bei 37 °C stimuliert.
Die Reaktion wird durch 1 % Ameisensäure gestoppt, der Überstand der Zellen mit 2 x 4 ml Ethylacetat extrahiert und dann mittels Hochdruckflüssigkeitschromatographie aufgetrennt. Das Ergebnis dieses Versuches zeigt Tabelle III:

Tabelle III

| | Konzentration der Verbindungen | | | | | | |
|---|---|---|---|---|---|---|---|
| Hemmung der 5-Lipoxygenase-aktivität | 0 | 1 | 2,5 | 5 | 10 | 20 | 30 µmol |
| (% Hemmung) | 0 | 24 | 40 | 100 | 100 | 100 | 100 |

Wie die Tabelle III zeigt, hemmen Konzentrationen der Verbindungen über 5 µM die Lipoxygenase von Schweine-Leukozyten zu 100 %. Damit wird die Entstehung von Leukotrienen völlig inhibiert.

Außer den in den Beispielen genannten Verbindungen sind Gegenstand der vorliegenden Erfindung insbesondere alle Substanzen, die jede mögliche Kombination der in den Beispielen genannten Substituenten aufweisen.

## Patentansprüche

**1.** Verwendung von Thiosulfinsäurederivaten der allgemeinen Formel (I)

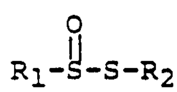

(I)

in der $R_1$ und $R_2$, die gleich oder verschieden sein können, einen gegebenenfalls einfach oder mehrfach substituierten Alkylrest, Arylrest, Aralkylrest, alicyclischen Rest oder heterocyclischen Rest bedeuten, zur Herstellung von Arzneimitteln zur Behandlung von entzündlichen Erkrankungen.

**2.** Verwendung einer Verbindung nach Anspruch 1 , worin $R_1$ und/oder $R_2$ gegebenenfalls substituiertes Phenyl bedeutet.

**3.** Verwendung einer Verbindung nach Anspruch 1, worin die Verbindung eine der folgenden Formeln aufweist:

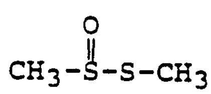

(II)

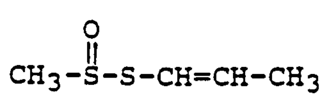

(trans)

(III)

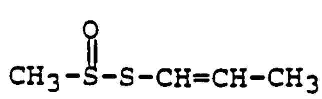

(cis)

(IV)

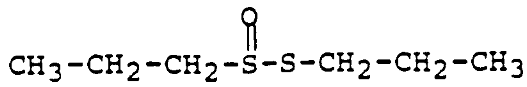

(V)

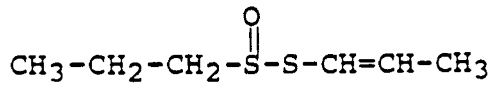

(cis)

(VI)

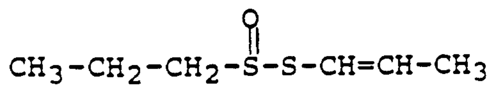

(trans)

(VII)

**4.** Verfahren zur Herstellung einer der Verbindungen (I) bis (VII) nach Anspruch 3, bei dem man

(i) Zwiebeln (Allium cepa) homogenisiert und nach Stehen bei Raumtemperatur daraus Zwiebelpreßsaft oder ein Lyophilisat gewinnt,

(ii) den Zwiebelpreßsaft bzw. das Lyophilisat im ersteren Fall mit einem polaren, mit Wasser nicht mischbaren Lösungsmittel bzw. im letzteren Fall mit einem niederen Alkohol oder unter Druck mit einem superkritischen Gas extrahiert

und

(iii) den Extrakt nach an sich bekannten Trenn-verfahren in seine Bestandteile auftrennt.

**5.** Verfahren nach Anspruch 4 bei dem man

(i) Zwiebeln (Allium cepa) homogenisiert, das Homogenat mindestens 20 min bei Raumtemperatur stehen läßt und anschließend daraus Zwiebelpreßsaft gewinnt,

(ii) den Zwiebelpreßsaft zweimal mit Dichlormethan, Chloroform oder anderen mit Wasser nicht mischbaren organischen Lösungsmitteln ausschuttelt, die organische Phase über Natriumsulfat trocknet und das Lösungsmittel unter vermindertem Druck abdestilliert;

(iii) den Rückstand durch Säulenchromatographie über Kieselgel mit Toluol/Ethylacetat als Elutionsmittel grobfraktioniert,

(iv) den aktiven Bestandteil einer erneuten chromatographischen Trennung über Kieselgel unterwirft, wobei man als Elutionsmittel Dichlormethan oder Chloroform mit einer Beimengung von 1 - 5 % Aceton oder Ethylmethylketon oder Diethylether oder Laufmittelsysteme auf Basis von Kohlenwasserstoffen wie Heptan, Pentan oder Hexan mit Beimengungen von 5 - 10 % Aceton oder Ethylmethylketon verwendet.

**6.** Verfahren nach Anspruch 5, bei dem man den in Schritt (ii) erhaltenen Rückstand mittels Flash-Chromatographie an reversed-phase Material unter Verwendung eines hydrophilen Laufmittels grobfraktioniert und den aktiven Bestandteil anschließend durchMitteldruckflüssigkeitschromatographie (MPLC) über Kieselgel unter Verwendung eines der in Schritt (iv) in Anspruch 4 genannten Lösungen als Elutionsmittel auftrennt.

**7.** Pharmazeutische Zubereitung enthaltend mindestens eine der Verbindungen nach Anspruch 1 bis 3 zur Verwendung als entzündungshemmendes Mittel.

**8.** Verwendung von Thiosulfinsäurederivate der allgemeinen Formel I'

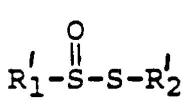

(I'),

in der

$R_1$' und $R_2$' gleich oder verschieden sein können und einen gegebenenfalls einfach oder mehrfach

substituierten Alkylrest, Arylrest, Aralkylrest, alicyclischen Rest oder heterocyclischen Rest bedeuten, wobei, wenn $R_1$' eine Allylgruppe bedeute, $R_2$' nicht gleichfalls eine Allylgruppe und, wenn $R_1$' eine Methylgruppe bedeutet, $R_2$' nicht gleichfalls eine Methylgruppe sein darf,
zur Herstellung von entzündungshemmenden Arzneimitteln.

**9.** Verwendung von Verbindungen der allgemeinen Formel I' gemäß Anspruch 8 zur Herstellung von Arzneimitteln für Behandlung des Bronchialasthmas.

**10.** Verwendung von Verbindungen der allgemeinen Formel I' gemäß Anspruch 8 zur Herstellung von Arzneimitteln für Behandlung PAF induzierter Entzündungszustände.

**11.** Verwendung von Verbindungen der allgemeinen Formel I' gemäß Anspruch 8 zur Herstellung von Arzneimitteln für Behandlung von Erkrankungen des rheumatischen Formenkreises, wie Polyarthritis.

**12.** Thiosulfinsäure-Derivate der allgemeinen Formel I''

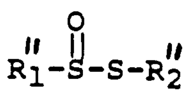

(I''),

in der
$R_1$'' einen gegebenenfalls einfach oder mehrfach substituierten Alkylrest, Arylrest, Aralkylrest, alicyclischen Rest, oder heterocyclischen Rest bedeuten
und
$R_2$'' einen gegebenenfalls substituierten $\alpha$, $\beta$-ungesattigten Alkylrest bedeuten.

**13.** Thiosulfinsäurederivate der Formeln

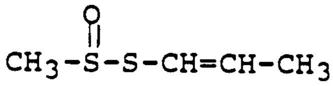 (III)

(trans)

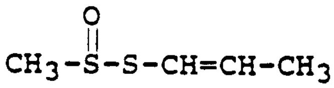 (IV)

(cis)

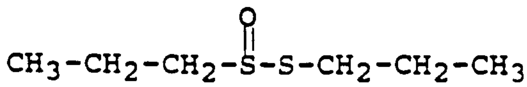 (V)

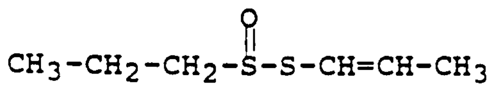 (VI)

(cis)

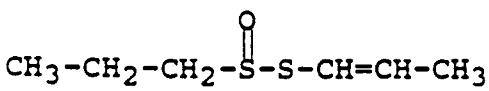 (VII)

(trans).

## Claims

**1.** Use of thiosulphinic acid derivatives of the general formula (I)

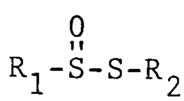 (I)

in which $R_1$ and $R_2$, which can be the same or different, signify a possibly singly or multiple substituted alkyl radical, aryl radical, aralkyl radical, alicyclic radical or heterocyclic radical, for the preparation of medicaments for the treatment of inflammatory diseases.

**2.** Use of a compound according to claim 1, wherein $R_1$ and/or $R_2$ signifies possibly substituted phenyl.

**3.** Use of a compound according to claim 1, wherein the compound displays one of the following formulae:

$$CH_3-\overset{O}{\overset{\|}{S}}-S-CH_3 \qquad (II)$$

$$CH_3-\overset{O}{\overset{\|}{S}}-S-CH=CH-CH_3 \qquad (III)$$

(trans)

$$CH_3-\overset{O}{\overset{\|}{S}}-S-CH=CH-CH_3 \qquad (IV)$$

(cis)

$$CH_3-CH_2-CH_2-\overset{O}{\overset{\|}{S}}-S-CH_2-CH_2-CH_3 \qquad (V)$$

$$CH_3-CH_2-CH_2-\overset{O}{\overset{\|}{S}}-S-CH=CH-CH_3 \qquad (VI)$$

(cis)

$$CH\ -CH_2-CH_2-\overset{O}{\overset{\|}{S}}-S-CH=CH-CH_3 \qquad (VII)$$

(trans)

**4.** Process for the preparation of one of the compounds (I) to (VII) according to claim 3, in which one
(i) homogenises onions (Allium cepa) and, after standing at room temperature, obtains therefrom pressed onion juice or a lyophilisate,
(ii) extracts the pressed onion juice or the lyophilisate in the first case with a polar solvent not miscible with water or in the latter case with a lower alcohol or under pressure with a super-critical gas and
(iii) separates the extract into its components according to per se known separation processes.

**5.** Process according to claim 4 in which one
(i) homogenises onions (Allium cepa), leaves the homogenate to stand at room temperature for at least 20 min and subsequently obtains pressed onion juice therefrom,
(ii) shakes out the pressed onion juice twice with dichloromethane, chloroform or other organic solvent not miscible with water, dries the organic phase over sodium sulphate and distils off the solvent under reduced pressure,
(iii) roughly fractionates the residue by column chromatography over silica gel with toluene/ethyl acetate as elution agent,
(iv) subjects the active component to a renewed chromatographic separation over silica gel, whereby, as elution agent, one uses dichloromethane or chloroform with an admixture of 1 - 5% acetone or ethyl methyl ketone or diethyl ether or elution agent systems based on hydrocarbons, such as heptane, pentane or hexane, with admixtures of 5 - 10% acetone or ethyl methyl ketone.

**6.** Process according to claim 5, in which one roughly fractionates the residue obtained in step (ii) by means of flash chromatography on reversed phase material with the use of a hydrophilic elution agent and subsequently separates the active component by means of medium pressure liquid chromatography (MPLC) over silica gel with the use of one of the solutions mentioned in step (iv) in claim 4 as elution agent.

7. Pharmaceutical composition containing at least one of the compounds according to claim 1 to 3 for use as inflammation-inhibiting agent.

8. Use of thiosulphinic acid derivatives of the general formula I'

$$R'_1-\overset{O}{\overset{\|}{S}}-S-R'_2 \qquad (I')$$

in which $R'_1$ and $R'_2$ can be the same or different and signify a possibly singly or multiple substituted alkyl radical, aryl radical, aralkyl radical, alicyclic radical or heterocyclic radical, whereby, when $R'_1$ signifies an allyl group, $R'_2$ cannot also be an allyl group and when $R'_1$ signifies a methyl group, $R'_2$ cannot also be a methyl group, for the preparation of inflammation-inhibiting agents.

9. Use of compounds of the general formula I' according to claim 8 for the preparation of medicaments for the treatment of bronchial asthma.

10. Use of compounds of the general formula I' according to claim 8 for the preparation of medicaments for the treatment of PAF-induced inflammatory conditions.

11. Use of compounds of the general formula I' according to claim 8 for the preparation of medicaments for the treatment of diseases of the rheumatic type, such as polyarthritis.

12. Thiosulphinic acid derivatives of the general formula I''

$$R''_2-\overset{O}{\overset{\|}{S}}-S-R''_2 \qquad (I'')$$

in which $R''_1$ signifies a possibly singly or multiple substituted alkyl radical, aryl radical, aralkyl radical, alicyclic radical or heterocyclic radical and $R''_2$ a possibly substituted $\alpha,\beta$-unsaturated alkyl radical.

**13.** Thiosulphinic acid derivatives of the formulae

$$CH_3-\overset{O}{\overset{\|}{S}}-S-CH=CH-CH_3 \quad \text{(III)}$$
(trans)

$$CH_3-\overset{O}{\overset{\|}{S}}-S-CH=CH-CH_3 \quad \text{(IV)}$$
(cis)

$$CH_3-CH_2-CH_2-\overset{O}{\overset{\|}{S}}-S-CH_2-CH_2-CH_3 \quad \text{(V)}$$

$$CH_3-CH_2-CH_2-\overset{O}{\overset{\|}{S}}-S-CH=CH-CH_3 \quad \text{(VI)}$$
(cis)

$$CH_3-CH_2-CH_2-\overset{O}{\overset{\|}{S}}-S-CH=CH-CH_3 \quad \text{(VII)}$$
(trans)

## Revendications

**1.** Utilisation de dérivés de l'acide thiosulfinique de formule générale (I)

$$R_1-\overset{O}{\overset{\|}{S}}-S-R_2 \quad \text{(I)}$$

dans laquelle $R_1$ et $R_2$, qui peuvent être identiques ou différents, représentent un groupe alkyle, aryle, aralkyle, alicyclique ou hétérocyclique, portant éventuellement un ou plusieurs substituants, pour la préparation de médicaments destinés au traitement de maladies à caractère inflammatoire.

**2.** Utilisation d'un composé selon la revendication 1, dans laquelle $R_1$ et/ou $R_2$ représentent un groupe phényle éventuellement substitué.

**3.** Utilisation d'un composé selon la revendication 1, dans laquelle le composé répond à une des formules suivantes :

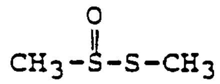
(II)

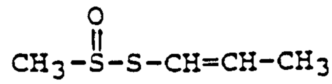
(trans)
(III)

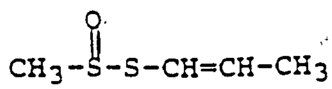
(cis)
(IV)

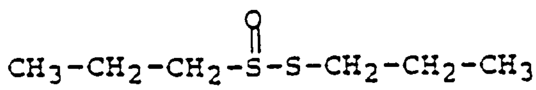
(V)

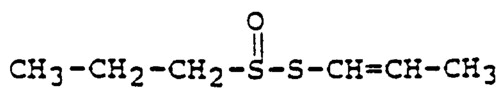
(cis)
(VI)

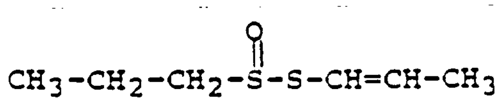
(trans)
(VII)

**4.** Procédé de préparation d'un des composés (I) à (VII), selon la revendication 3, dans lequel
(i) on homogénéise des oignons (Allium cepa) et, après repos à la température ambiante, on en obtient du jus d'oignon ou un lyophilisat,
(ii) on extrait le jus d'oignon ou le lyophilisat, dans le premier cas avec un solvant polaire, non miscible à l'eau, et dans le deuxième cas, avec un alcool inférieur ou sous pression avec un gaz supercritique, et
(iii) on sépare les constituants de l'extrait par des procédés de séparation connus en soi.

**5.** Procédé selon la revendication 4, dans lequel
(i) on homogénéise des oignons (Allium cepa), on laisse l'homogénéisat au repos pendant au moins 20 minutes et ensuite, on le transforme en jus d'oignon,
(ii) on extrait le jus d'oignon deux fois avec du dichlorométhane, du chloroforme ou d'autres solvants organiques non miscibles a l'eau, on sèche la phase organique sur du sulfate de sodium et l'on élimine le solvant par distillation sous pression réduite ;
(iii) on soumet le résidu à un fractionnement grossier par chromatographie sur colonne, sur du gel de silice, avec comme éluant du toluène/acétate d'éthyle,
(iv) on soumet ce composant actif à une nouvelle séparation chromatographique sur du gel de silice, en utilisant comme éluant du dichlorométhane ou du chloroforme, contenant une proportion d'acétone ou d'éthylméthylcétone ou de diéthyléther de 1-5 %, ou un éluant à base d'hydrocarbure comme l'heptane, le pentane ou l'hexane, contenant une proportion d'acétone ou de diméthylcétone de 5-10 %.

6. Procédé selon la revendication 5, dans lequel on soumet le résidu obtenu dans l'étape (ii) à un fractionnement grossier par chromatographie éclair sur un matériau de séparation en phases inversées, en utilisant un éluant hydrophile, et l'on sépare ensuite le composant actif par chromatographie en phase liquide moyenne pression (MPLC) sur du gel de silice, en utilisant comme éluant l'un des solvants mentionnés dans l'étape (iv) de la revendication 4.

7. Préparation pharmaceutique contenant au moins un des composés selon les revendications 1 à 3, pour application comme agent anti-inflammatoire.

8. Utilisation de dérivés de l'acide thiosulfinique de formule générale I'

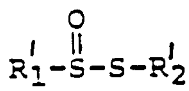

(I'),

dans laquelle $R_1$' et $R_2$', qui peuvent être identiques ou différents, représentent un groupe alkyle, aryle, aralkyle, alicyclique ou hétérocyclique, portant éventuellement un ou plusieurs substituants,
dans laquelle, lorsque $R_1$' représente un groupe allyle, $R_2$' ne peut pas représenter également un groupe allyle, et, lorsque $R_1$' représente un groupe méthyle, R2' ne peut pas représenter également un groupe méthyle,
pour la préparation d'un médicament anti-inflammatoire.

9. Utilisation de composés répondant à la formule générale I' selon la revendication 8, pour la préparation de médicaments destinés au traitement de la bronchite asthmatiforme.

10. Utilisation de composés répondant à la formule générale I' selon la revendication 8, pour la préparation de médicaments destinés au traitement d'états inflammatoires provoqués par le facteur activateur des plaquettes (PAF).

11. Utilisation de composés répondant à la formule générale I' selon la revendication 8, pour la préparation de médicaments destinés au traitement d'affections du type rhumatismale, telles que la polyarthrite.

12. Dérivés de l'acide thiosulfinique de formule générale I''

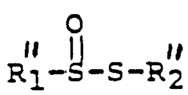

(I''),

dans laquelle $R_1$'' représente un groupe alkyle, aryle, aralkyle, alicyclique ou hétérocyclique, portant éventuellement un ou plusieurs substituants,
et
$R_2$'' représente un groupe alkyle $\alpha,\beta$-insaturé, éventuellement substitué.

**13.** Dérivés de l'acide thiosulfinique de formule

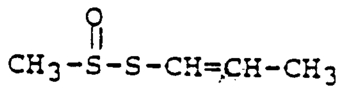

(trans) (III)

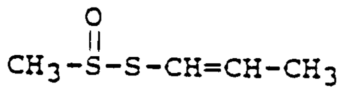

(cis) (IV)

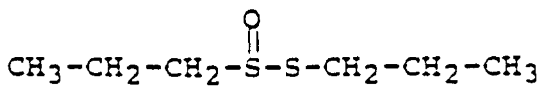

(V)

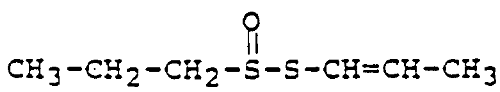

(cis) (VI)

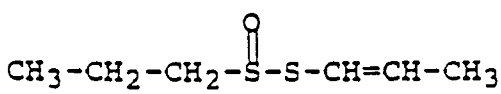

(trans). (VII)

Abbildung 1

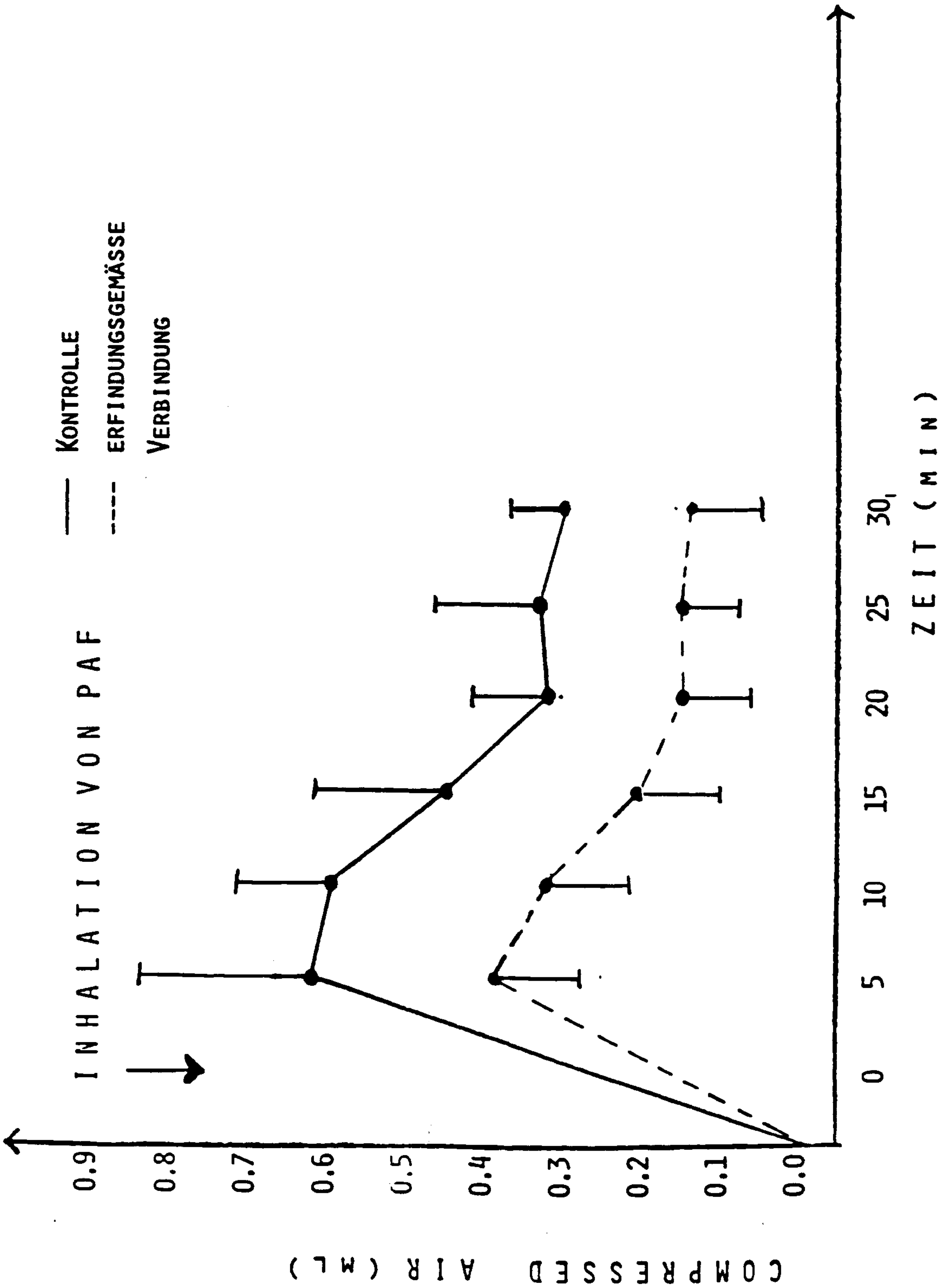
INHALATION VON PAF
KONTROLLE
ERFINDUNGSGEMÄSSE VERBINDUNG
COMPRESSED AIR (ML)
0.0
0.1
0.2
0.3
0.4
0.5
0.6
0.7
0.8
0.9
ZEIT (MIN)
0
5
10
15
20
25
30

Abbildung 2

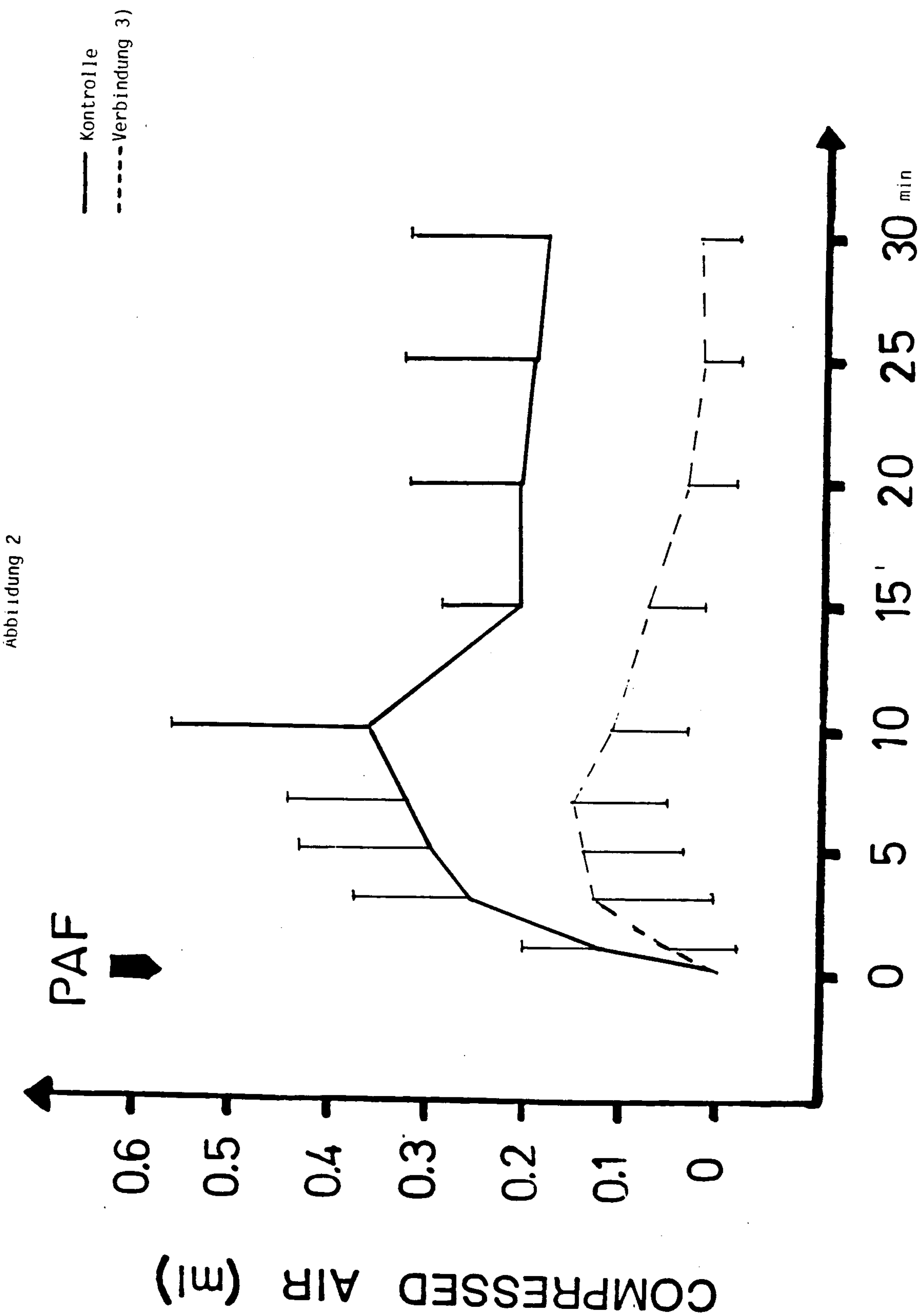
PAF
Kontrolle
Verbindung 3)
COMPRESSED AIR (ml)
0.6
0.5
0.4
0.3
0.2
0.1
0
0
5
10
15'
20
25
30 min

Abbildung 3

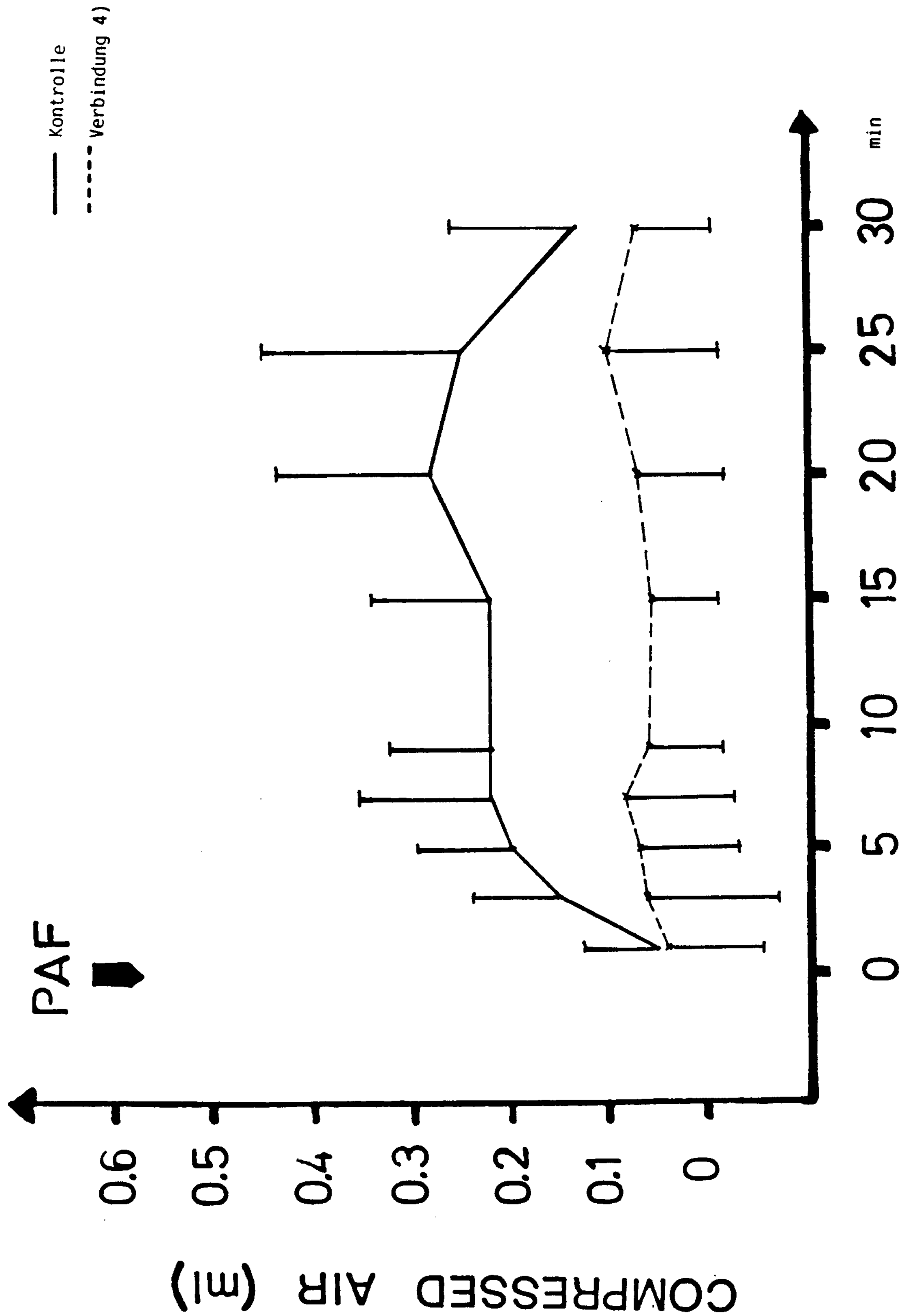
Kontrolle
Verbindung 4)
PAF
COMPRESSED AIR (ml)
0.6
0.5
0.4
0.3
0.2
0.1
0
0
5
10
15
20
25
30
min

Abbildung 4

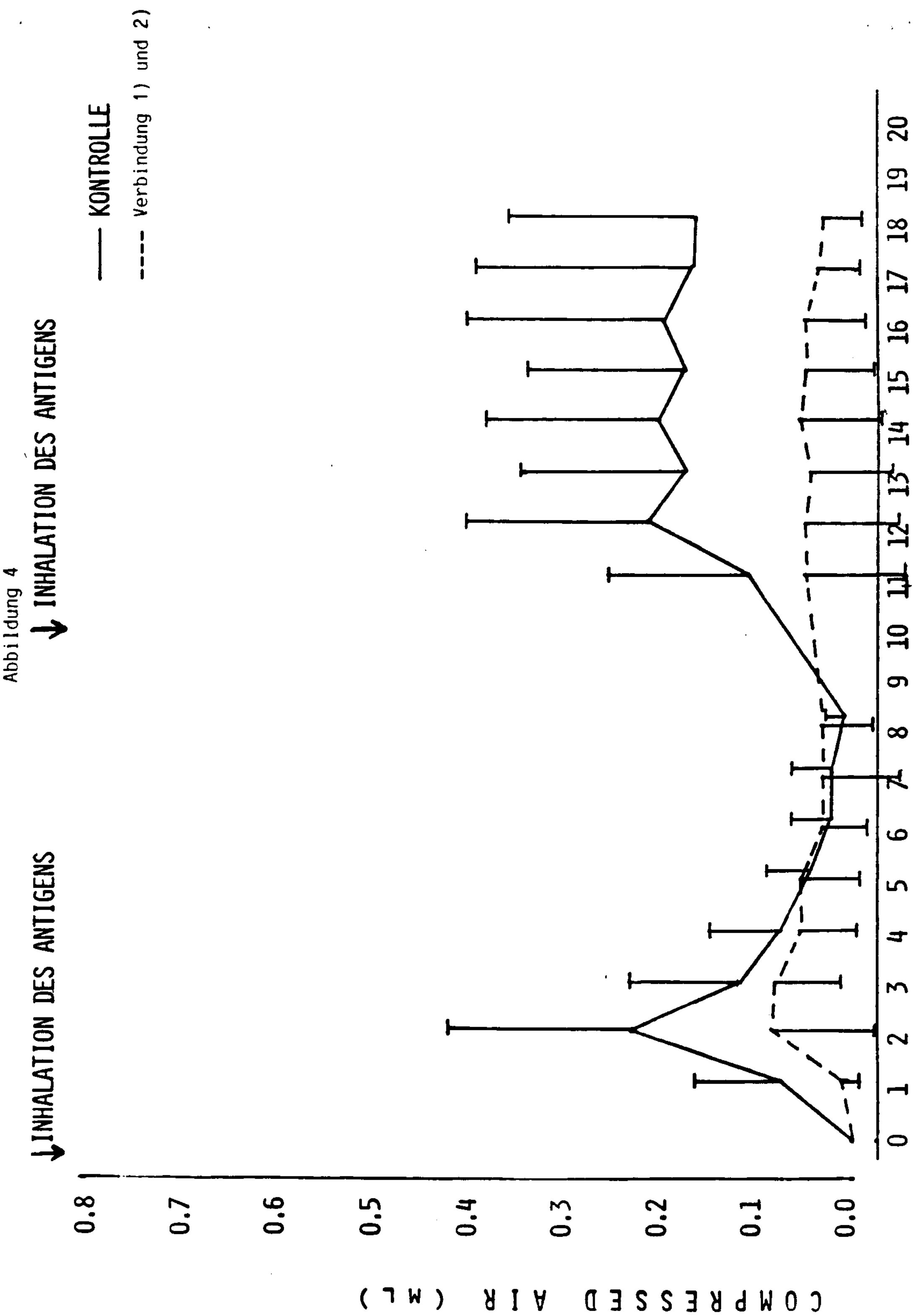


ABBILDUNG 2

Abbildung 5

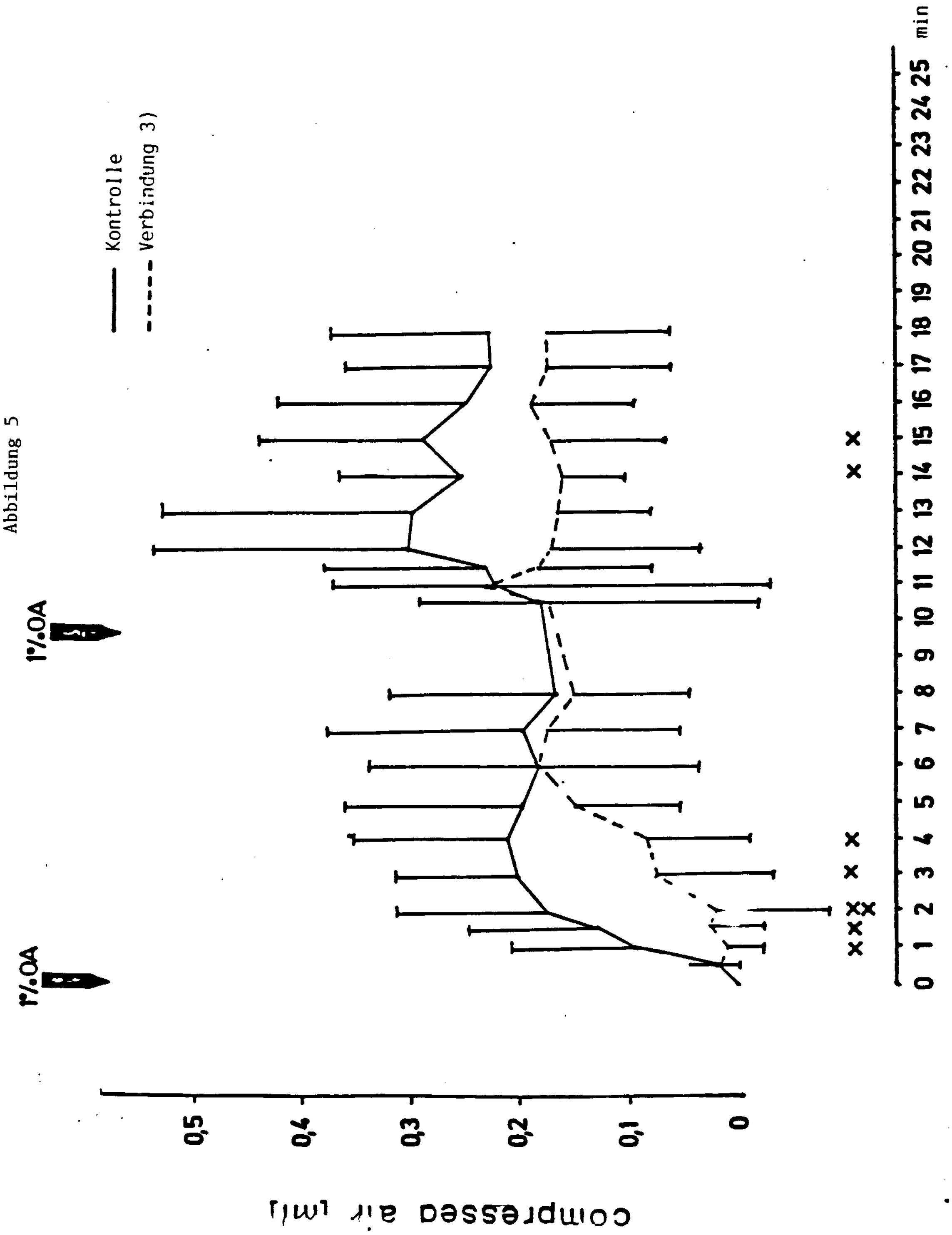
1%OA
1%OA
Kontrolle
Verbindung 3)
compressed air (ml)
0,5
0,4
0,3
0,2
0,1
0
0 1 2 3 4 5 6 7 8 9 10 11 12 13 14 15 16 17 18 19 20 21 22 23 24 25
min

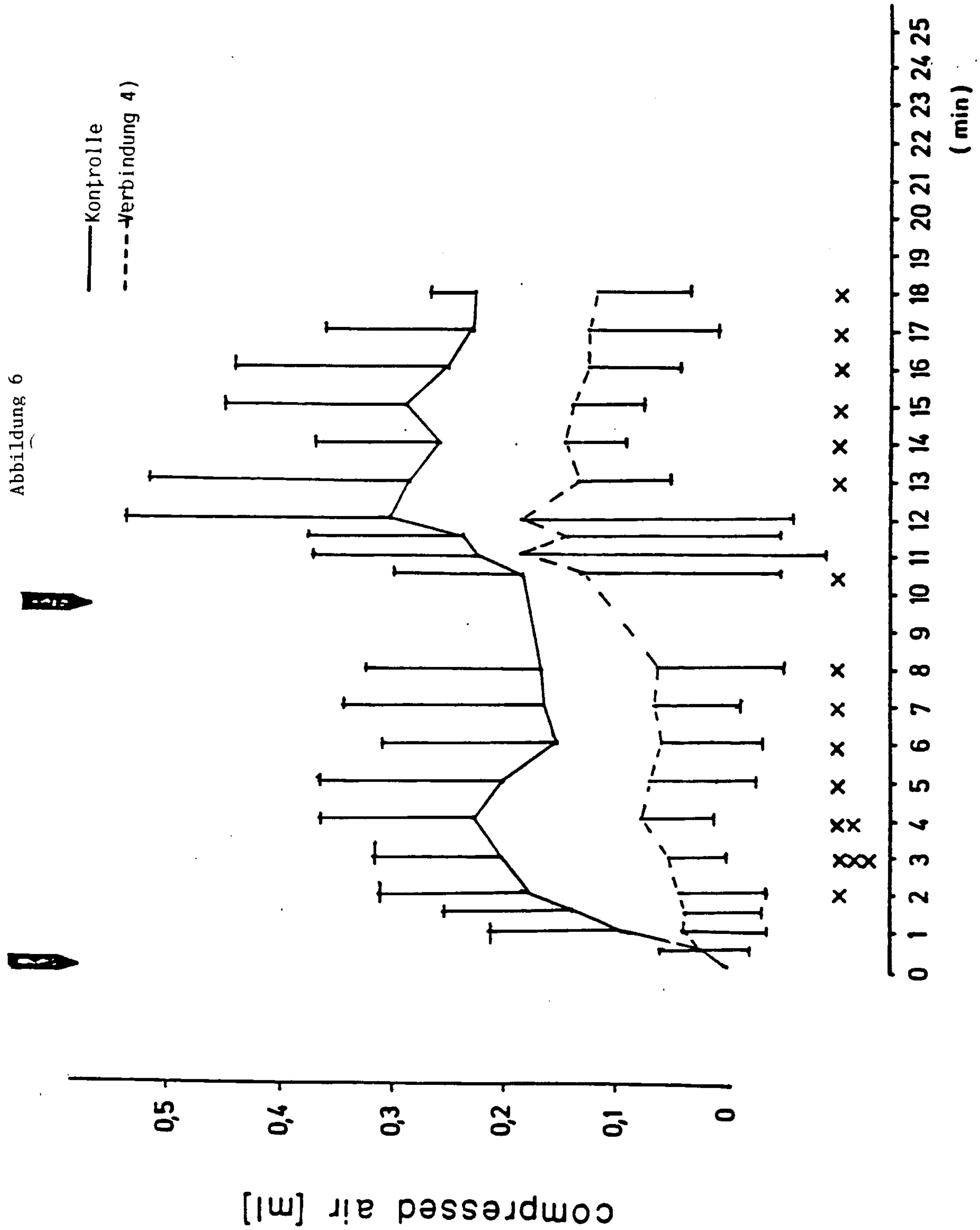
Abbildung 6
Kontrolle
Verbindung 4)
compressed air [ml]
0,5
0,4
0,3
0,2
0,1
0
0 1 2 3 4 5 6 7 8 9 10 11 12 13 14 15 16 17 18 19 20 21 22 23 24 25
(min)